# EUROPEAN PATENT APPLICATION

(11) **EP 1 900 736 A1**
(43) Date of publication of application: **19.03.2008**
(21) Application number: 07150376.7
(22) Date of filing: 19.11.2002
(51) Int. Cl.: C07D 417/12

(54) **5-[4-[2-(n-methyl-n-(2-pyridyl)amino)ethoxy]benzyl]thiazolidine-2,4-dione and pharmaceutical compositions comprising the same**

(30) Priority: 20.11.2001 GB 0127805
(62) Divisional of application: 02779696.0
(71) Applicant: SMITHKLINE BEECHAM PLC, Brentford, Middlesex TW8 9GS (GB)
(72) Inventor: Leonard, Graham, Stanley, Harlow, Essex CM19 5AW (GB); Lewis, Karen, Jane, I-37135, Verona (IT); Mackenzie, Donald, Colin, Harlow, Essex CM19 5AW (GB)
(74) Representative: Rutter, Keith

(57) **Abstract**

A compound, 5-[4-[2-(N-methyl-N-(2-pyridyl)amino)ethoxy]benzyl]thiazolidine-2,4-dione ('Compound (I)'), or a pharmaceutically acceptable form thereof, in particulate form wherein the median value of the volume mean diameter of the particles is within the range 500nm to 5 micrometres and medicinal uses thereof.

## Description

This invention relates to novel particulate forms of certain compounds, compositions comprising such compounds and the use of such novel partculate forms and compositions in therapy.

The dry milling of pharmaceutically active compounds to obtain a particle size appropriate for tablet and other formulations is well known in the art. In particular, air j et milling and fluid energy milling (micronising) are favoured because of the reduced risk of contamination.

Wet milling processes have also been disclosed for the preparation of finely divided particles of drug substance to enhance bioavailability. For example, United States Patent 4,540,602 (Freund Industry Company Limited) discloses a process for the preparation of finely divided particles of a sparingly soluble drug substance by dispersal of the drug substance in water in the presence of a substance of high molecular weight and then removing the water from the disperse system. European Patent 0 499 299 (NanoSystems L.L.C) discloses a wet milling procedure for the production of particles of a crystalline drug substance, which particles have a surface modifier adsorbed on the surface in an amount sufficient to maintain an effective average particle size (95 to 99% below) of less than about 400nm. International Patent Application Publication Number WO 99/30687 (SmithKline Beecham PLC) discloses pharmaceutical compositions ofbicyclic drug substances, which compositions have a particle size distribution wherein the median value of the volume mean diameter is within the range 350 to 700nm. WO 01/13889 (SmithKline Beecham PLC) discloses pharmaceutical compositions of nabumetone, which compositions have a particle size distribution of nabumetone such that the median particle size is in the range of about 10 micrometres to about 0.55 micrometres. WO 00/18374 (Elan Pharma International Ltd.) discloses controlled release compositions containing a poorly-soluble agent such as a drug.

European Patent 0 306 228 (Beecham Group PLC) relates to certain thiazolidinedione derivatives disclosed as having hypoglycaemic and hypolipidaemic activity. One particular thiazolidinedione disclosed in EP 0 306 228 is 5-[4-[2-(N-methyl-N-(2-pyridyl)amino)ethoxy]benzyl]thiazolidine-2,4-dione (hereinafter referred to as 'Compound (I)'). European Patent 0 658 161 (SmithKline Beecham PLC) discloses certain salts of Compound (I), including the maleate salt at Example 1 thereof.

It is considered that Compound (I) and pharmaceutically acceptable forms thereof have enhanced bioavailability in certain compositions of controlled particle size.

It is also considered that certain compositions of therapeutic agents and pharmaceutically acceptable forms thereof of controlled particle size are particularly advantageous for the preparation of controlled release compositions. Such compositions are particularly useful for; selecting the site of delivery of the therapeutic agent in the human or animal body, for example to avoid adverse physiological responses such as stomach irritation; and for controlling the release of the therapeutic agent to select the desired pharmacokinetic profile, for example to optimise the therapeutic effect.

Accordingly, in a first aspect, the present invention provides Compound (I), or a pharmaceutically acceptable form thereof, in particulate form wherein the median value of the volume mean diameter of the particles is in the range of from 500nm to 5 micrometres, typically less than 1 micrometre.

Thus, suitable particle sizes are those wherein the median value of the volume mean diameter of the particles is within the range of500nm to 5 micrometres;

Suitable particle sizes also include those wherein the median value of the volume mean diameter of the particles is within the range of from 600nm to 5 micrometres, 700nm to 5 micrometres, 800nm to 5 micrometres, 900nm to 5 micrometres or 1000nm to 5 micrometres.

Suitable particles further include those having a median value of the volume mean diameter of the particles in the range of from: 500nm to 4.5 micrometres, 500nm to 4.0 micrometres micrometres, 500nm to 3.5 micrometres, 500nm to 3.0 micrometres, 500nm to 2.5 micrometres, 500nm to 2.0 micrometres, 500nm to 1.5 micrometres or 500nm to 1.0 micrometre. One such particle size is 500nm to 4.5 micrometres. One such particle size is 500nm to 4.0 micrometres micrometres,. One such particle size is 500nm to 3.5 micrometres. One such particle size is 500nm to 3.0 micrometres. One such particle size is 500nm to 2.5 micrometres. One such particle size is 500nm to 2.0 micrometres. One such particle size is 500nm to 1.5 micrometres. One such particle size is 500nm to 1.0 micrometre.

A preferred particle size is that wherein the median value of the volume mean diameter of the particles is in the range of from 500nm to 1000nm.

Particle sizes are measured using conventional particle size measuring techniques, such as light scattering techniques.

The particulate form of Compound (I), or a pharmaceutically acceptable form thereof, is most suitably prepared by wet milling as an aqueous dispersion. The wet milling is preferably carried out using a suitable milling medium in one, two or a plurality of bead mill chambers with a single pass sequentially through the chambers. To reduce contamination from mill materials, the milling medium is preferably ceramic beads of rare earth oxides and is carried out in chambers lined with or constructed from an abrasion-resistant polymer material such as polyamide (e.g. Nylon). If a bead mill of more than one chamber is used, then each chamber may contain beads of a smaller diameter than those in the previous chamber. For example, if a two chamber mill is used, then the beads in the first chamber are suitably of 1.25 mm diameter, and the beads in the second chamber are of 0.4 mm diameter. The milling medium is suitably beads made of zirconia, preferably yttria-stabilized zirconia powder.

To assist in further processing, that is the preparation of pharmaceutical formulations for therapeutic use, such as tablets, and injectable dispersions, the wet milling preferably takes place in an aqueous medium, where necessary including one or more excipients such as a soluble carrier suitable for spray drying, a surfactant and/or a stabiliser to maintain the particles in suspension, and an anti-agglomeration agent effective after administration of the pharmaceutical formulation to a patient.

In the aqueous medium to be subjected to the milling, Compound (I), or a pharmaceutically acceptable form thereof, may be present from about 5 to about 50% w/w. At 30% w/w and above there may be difficulties in maintaining a suspension of the Compound (I), or a pharmaceutically acceptable form thereof, during milling, hence suspending and/or stabiliser may be required.

A suitable surfactant is sodium lauryl sulphate.

Suitably, the surfactant or stabiliser is present in the range from about 0.1 to about 0.5% w/w of the aqueous medium.

Preferably the surfactant or stabiliser is present at about 1% by weight of the compound to be processed.

Suitable anti-agglomeration agents are cellulosic thickening agents such as hydroxypropyl methyl cellulose and hydroxyethyl cellulose.

Suitably, the anti-agglomeration agent is present in the range from about 1% w/w to about 2% w/w of the aqueous medium.

Preferably, the anti-agglomeration agent is present at about 1.5% w/w of the aqueous medium.

A suitable combined anti-agglomeration agent and suspending agent is polyvinylpyrrolidone.

Suitably, the combined anti-agglomeration agent and suspending agent is present at in the range from about 0.5% w/w to about 10% w/w of the aqueous medium.

Preferably, the anti-agglomeration agent and suspending agent is present at about 1.5% w/w of the aqueous medium.

Suitable pharmaceutically acceptable forms of Compound (I) include those described in EP 0 306 228 and EP 0 658 161, especially pharmaceutically acceptable salts or pharmaceutically acceptable solvates.

The suspension is buffered, suitably to a pH of 6.5, to reduce the solubility of Compound (I), or a pharmaceutically acceptable form thereof, during the milling process.

A preferred pharmaceutically acceptable salt of Compound (I) is the maleate salt.

A preferred pharmaceutically acceptable solvate of Compound (I) is a hydrate.

The free base of Compound (I) is particularly suitable for the process of the invention due to its reduced aqueous solubility in comparison to the salts and solvates.

As previously discussed herein, the wet milled Compound (I), or a pharmaceutically acceptable form thereof, may be used in the preparation of pharmaceutical compositions.

Accordingly, in a further aspect, there is provided a composition, suitably a pharmaceutically acceptable composition, comprising a Compound (I), or a pharmaceutically acceptable form thereof, in particulate form, wherein the median value of the volume mean diameter of the particles is in the range of from 500nm to 5 micrometres, typically less than 1 micrometre.

Suitable particle sizes are as mentioned above. One preferred particle size is less than 1 micrometre.

The aqueous dispersion obtained from the milling process may be used directly as a therapeutic agent if prepared under conditions of appropriate hygiene. Using water and other components which meet Ph Eur standards However, for the preparation of formulations for use in human therapy, it is a preferred method that the aqueous dispersion is converted to a dried powder. This is carried out most suitably by spray drying, typically collecting the product from the dryer using a cyclone separator.

Additional excipients may be added to the milled aqueous suspension to produce a suspension suitable for the spray drying process. Examples of such additional excipients include carriers such as mannitol, sorbitol, lactose, lactitol, xylitol, and starch. A powder composition containing the particulate Compound (I), or a salt thereof, is obtainable as a composition which has good flowability and is suitable for incorporation into a tablet formulation or a powder formulation for capsules.

A composition of this invention may be in the form of an aqueous dispersion of the particles of Compound (I), or a pharmaceutically acceptable form thereof, typically the product of a wet milling operation. A composition of this invention may also be a powder comprising particles of a Compound (I), or a pharmaceutically acceptable form thereof, typically the product of a spray drying or spray granulation procedure suitably prepared from the particles mentioned above.

As previously discussed herein, certain compositions of therapeutic agents and pharmaceutically acceptable forms thereof of controlled particle size are particularly advantageous for the preparation of controlled release compositions.

Accordingly, in a further aspect, the invention provides a controlled release pharmaceutical composition, wherein the therapeutic agent or a pharmaceutically acceptable form thereof of controlled particle size and a pharmaceutically acceptable carrier are combined, wherein the carrier is selected to provide a controlled release of the therapeutic agent, or a pharmaceutically acceptable form thereof.

In another aspect the invention provides a controlled release pharmaceutical composition, comprising a therapeutic agent and a carrier, the therapeutic agent having a controlled particle size and wherein the carrier is selected to provide a controlled release of the therapeutic agent.

The content of the therapeutic agent in the spray dried product is typically in the range of 2 to 90 % w/w.

Suitably, the therapeutic agent is Compound (I), or a pharmaceutically acceptable form thereof.

The milled suspension is mixed with polymers, plasticisers and glidants where appropriate and spray dried to produce a multiparticulate controlled release product. The controlled release product may suitably be filled into capsules or compressed into a disintegrating tablet formulation.

The controlled release dosage form is a multiparticulate dosage form, where the release rate from the dosage form, and transit through the gastrointestinal tract is independent of the presence of food and the time of dosing.

As used herein, the term "controlled release" means a composition which has been designed to produce a desired pharmacokinetic profile by choice of appropriate form of the active ingredient and/or by choice of formulation. An example of a controlled release formulation is a modified release formulation. Controlled release also includes controlled release compositions in combination with non-controlled release compositions.

As used herein "controlled particle size" means particles having sizes as defined herein.

As used herein, the term "modified release" means a composition which has been designed to produce a desired pharmacokinetic profile by choice of formulation. Modified release also includes modified release compositions in combination with non-modified release compositions.

Examples of modified release include delayed, pulsed, and sustained release, either alone or in any combination.

Delayed release may conveniently be obtained by use of a gastric resistant formulation such as an enteric formulation, such as a granule incorporating a gastric resistant polymer, for example Eudragit L100-55. Other gastric resistant polymers include methacrylates, cellulose acetate phthalate, polyvinyl acetate phthalate, hydroxypropyl methylcellulose phthalate, in particular, Aquateric, Sureteric, HPMCP-HP-55S.

Sustained release means providing release of the active agent over an extended period of time, for example up to 24 hours, typically 4 to 24 hours.

Sustained release is typically provided by the incorporation of a disintegrating, non-disintegrating or eroding polymer into the spray dried product.

Sustained release is suitably obtained by use of a non-disintegrating formulation, for example by incorporating Eudragit RS into the spray dried product. Alternative non disintegrating matrix formulations are provided by incorporating methacrylates, cellulose acetates, hydroxypropyl methylcellulose phthalate, in particular Eudragit L and RL , Carbopol 971P, HPMCP-HP-55S into the formulation.

Sustained release can also be achieved by incorporating a semi-permeable polymer into the spray dried product for example methacrylates, ethylcellulose, cellulose acetate in particular Eudragit RS, or Surelease to the formulation.

Pulsed release means providing doses of the active agent in pulses, for example providing up to 4, for example 2, pulses of active agent per 24 hours.

One form of pulsed release is a combination of non-modified release of active agent and delayed release.

Suitably, a controlled release composition is a modified release composition.

Suitably, the modified release composition is a delayed release composition.

Suitably, the modified release composition is a sustained release composition.

Suitably, the modified release composition is a pulsed release composition.

Suitably, the composition of the invention is a combined non-modified and modified release composition. One example of such a combination provides non-modified and sustained release of active agent, for example non-modified release of 2mg of Compound (I), or a pharmaceutically acceptable form thereof, and sustained release of 8mg of Compound (I) or a pharmaceutically acceptable form thereof, over a 12 hour period; or non-modified release of 2mg of Compound (I), or a pharmaceutically acceptable form thereof, and sustained release of 6mg of Compound (I), or a pharmaceutically acceptable form thereof, over a 12 hour period; or non-modified release of 1mg of Compound (I), or a pharmaceutically acceptable form thereof, and sustained release of 7mg of Compound (I), or a pharmaceutically acceptable form thereof, over an 18 hour period.

The administration of the therapeutic agent, or a pharmaceutically acceptable form thereof, in particulate form to a mammal may be by way of oral, parenteral, buccal, sub-lingual, nasal, pulmonary, ocular, vaginal, rectal or transdermal administration. Transdermal administration includes sprays, patches, creams, and ointments.

A suitable dosage form for the spray dried controlled release product, is a tablet or capsule. The release of the therapeutic agent, or a pharmaceutically acceptable form thereof, can be further delayed by the addition of a coat to the tablet or capsule.

In particular the milled suspension of Compound (I) or a pharmaceutically acceptable form thereof is suitable for formulation for delivery by inhalation.

For example, the milled suspension is spray dried with a suitable carrier, for example lactose, with the particle size of the spray dried product being controlled to be within the range of 1 to 5 micrometres. The spray dried product may then be inhaled, using, for example, a suitable device such as a dry powder dispenser.

Alternatively, the milled suspension, without further processing, may be placed in a suitable pressurised device, for example a metered dose aerosol device, from which the product may be inhaled.

It is also considered that the inhaled formulations of Compound (I) or a pharmaceutically acceptable form thereof, are suitable for combination with insulin to provide a combination formulation for inhalation. These combination formulations may be in the form of a spray-dried product for inhalation as a dry powder, or for inhalation as a suspension from, for example, a metered dose inhaler.

Accordingly, there is provided a pharmaceutical composition comprising Compound (I) or a pharmaceutically acceptable form thereof in combination with insulin.

Suitably, the formulations for delivery by inhalation contain Compound (I) as the active ingredient.

Aerosol devices may be prepared using conventional methods well-known in the art. Formulations suitable for use in aerosol devices suitably include surfactants such as lecithin.

Suitable propellants for aerosol devices include chlorofluorocarbons such as trichloromethane, dichlorofluoromethane, and hydrofluoroalkane.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Tablets and capsules for oral administration are usually presented in a unit dose, and contain conventional excipients such as binding agents, fillers, diluents, tabletting agents (compression aids), lubricants, disintegrants, colorants, flavourings, and wetting agents. The tablets may be coated according to well known methods in the art.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, magnesium aluminium silicate, microcrystalline cellulose, methyl cellulose, polyvinylpyrrolidone, polacrilin potassium, pregelatinised starch, sodium alginate, sodium starch glycollate.

An example of a pharmaceutically acceptable wetting agent is sodium lauryl sulphate.

As required the solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice.

Compositions other than solid oral compositions may contain excipients such as carriers, formulation bases, surfactants, emulsifiers, and penetration enhancers, and are prepared using techniques known in the art.

Suitable carriers and formulation bases include water, liquid paraffin, white soft paraffin, mygliol, polyethylene glycol, glycerol, carbomer, hydroxypropylmethyl cellulose, and methyl cellulose.

Suitable surfactants and emulsifiers include cetostearyl alcohol and sodium lauryl sulphate.

Suitable penetration enhancers include ethanol, propylene glycol, oleic acid, decylmethyl sulphoxide, dimethyl sulphoxide, dimethyl acetamide, dimethyl formamide, 2-pyrrolidone, N-methyl-2-pyrrolidone, and azone.

Compositions may, if desired, be in the form of a pack accompanied by written or printed instructions for use.

A unit dose will normally contain 0.01 to 1000 mg of Compound (I) or a pharmaceutically acceptable form thereof..

Suitable unit doses for 1 to 100 mg, for example an amount in the range of from 2 to 50 mg such as 2, 4, 8, 10, and 12 mg of the active compound.

No adverse toxicological effects are expected for the compositions of the invention in the above mentioned dosage ranges.

The above mentioned publications, including published patent applications and patents, are incorporated herein by reference as though fully set forth herein.

The following Examples illustrate the invention but do not limit it in any way. Examples 1 and 2 are typical examples of a suspension for wet bead milling and spray drying of the milled suspension. Examples 3-7 are suitable formulations to produce a spray dried powder with modified release of Compound (I). Example 8 is a typical formulation for a compressed tablet produced from the spray dried material. The examples all produce a modified release dissolution profile (either in capsule or tablet form), where there is no or reduced drug release in an acid environment similar to that of the stomach, compared to an immediate release tablet, with subsequent release over 6-8 hours in a higher pH similar to that of the small intestine.

A suitable bead mill is the AP0010 mill from Nylacast Ltd., Leicester, UK. Bead mills manufactured by others such as Dena Systems BK Ltd., Barnsley, UK; Drais GmbH, Mannheim, Germany; or Netzsch GmbH, Selb, Germany could also be used for the wet milling drug substances.

A 500g batch of an aqueous suspension containing 10% w/w of compound 1 (for preparation see Example 1) was passed through a Dena DM-100 bead mill, a single chamber mill, in either a single pass or recirculation configuration, containing 85% by volume of yttrium stabilised 0.3mm zirconium oxide beads (from Tosoh, Japan). The chamber pressure during processing varied between 0.1 and 0.2MPa (1 and 2 bar; 14 and 28 psi). The yield exceeded 85%. The finely milled suspension was subsequently spray dried with addition of other excipients as required.

The milled suspensions were spray dried using a Mobile Minor Spray Dryer, manufactured by Niro Ltd, Denmark. In this apparatus, the aqueous suspension from the mill is fed by pump into a drying chamber as an atomised spray. The dried powder passes into a cyclone separator from which powdered product is removed. The spray dryer was operated in accordance with the manufacturers instructions at the following settings:

| | | |
|---|---|---|
| Machine Settings | Atomiser Speed | about 15,000 rpm |
| | Exhaust Fan/Heater | Setting II |
| | Pump Speed | 20 rpm |
| | Inlet Temperature | 85°C |
| | Outlet Temperature | 35°C |

### Examples

| 1. Suspension for Wet Bead Milling | |
|---|---|
| | %w/w |
| Compound (I) | 10 |
| Polyvinylpyrollidone | 1.5 |
| Purified Water buffered to pH 6.5 to | 100 |

Particle size as measured using conventional light scattering techniques, have D10 and D90 values, of 300nm to 3 micrometres respectively.

| 2. Suspension of Compound I for Spray Drying | |
|---|---|
| | %w/w |
| Milled Suspension of Compound I (as in Example 1) | 70 |
| Mannitol | 14 |
| Water to | 100 |

| 3. Suspension of Compound I + Polymer for Spray Drying | |
|---|---|
| | %w/w |
| Milled Suspension of Compound I (as in Example 1) | 12 |
| Eudragit L 30D (30% dispersion) | 44 |
| Triethyl Citrate | 0.5 |
| Talc | 0.5 |
| Water to | 100 |

| 4. Suspension of Compound I + Polymer for Spray Drying | |
|---|---|
| with reduced polymer content and no talc | %w/w |
| Milled Suspension of Compound I (as in Example 1) | 20 |
| Eudragit L 30D (30% dispersion) | 40 |
| Triethyl Citrate | 1.0 |
| Water to | 100 |

| 5. Suspension of Compound I + Polymer for Spray Drying | |
|---|---|
| with reduced polymer content and Mannitol | %w/w |
| Milled Suspension of Compound I (as in Example 1) | 26 |
| Eudragit L 30D (30% dispersion) | 37 |
| Mannitol | 0.5 |
| Water to | 100 |

| 6. Suspension of Compound I + Polymer for Spray Drying | |
|---|---|
| with combination of polymers | %w/w |
| Milled Suspension of Compound I (as in Example 1) | 17 |
| Eudragit L 30D (30% dispersion) | 32 |
| Eudragit NE (30% dispersion) | 8 |
| Water to | 100 |

| 7. Suspension of Compound I + Polymer for Spray Drying | |
|---|---|
| with a different polymer | %w/w |
| Milled Suspension of Compound I (as in Example 1) | 12 |
| Sureteric 15 % dispersion | 87 |
| Triethyl Citrate | 0.5 |
| Talc | 0.5 |
| Water to | 100 |

| 8. Disintegrating Modified Release Tablet | |
|---|---|
| Compression of spray dried material | mg/tablet |
| SD Granules (equivalent to 8mg pfb Compound I) | 102 |
| Microcrystalline Cellulose | 165 |
| Sodium starch glycolate | 30 |
| Magnesium stearate | 3 |

## Claims

1. A compound, 5-[4-[2-(N-methyl-N-(2-pyridyl)amino)ethoxy]benzyl]thiazolidine-2,4-dione ('Compound (I)'), or a pharmaceutically acceptable form thereof, in particulate form wherein the median value of the volume mean diameter of the particles is within the range 500nm to 5 micrometres.

2. A compound according to claim 1, wherein the median value of the volume mean diameter of the particles is less than 1 micrometre.

3. A compound according to claim 1 or claim 2, wherein the median value of the volume mean diameter of the particles is in the range of from 500nm to 1000nm.

4. A compound according to any one of claims 1 to 3, when prepared by wet milling, suitably as an aqueous dispersion.

5. A compound according to any one of claims 1 to 4, being the maleate salt of Compound (I).

6. A compound according to any one of claims 1 to 4, being Compound (I).

7. A pharmaceutical composition comprising a Compound (I), or a pharmaceutically acceptable form thereof, in particulate form, with a particle size of less than 1 micrometre.

8. A controlled release pharmaceutical composition, comprising Compound (I), or a pharmaceutically acceptable form thereof (the therapeutic agent) and a carrier, the therapeutic agent having a controlled particle size and wherein the carrier is selected to provide a controlled release of the therapeutic agent.
